# EUROPEAN PATENT APPLICATION

(11) **EP 1 674 061 A1**
(43) Date of publication of application: **28.06.2006**
(21) Application number: 04030646.6
(22) Date of filing: 23.12.2004
(51) Int. Cl.: A61F 11/14, H04R 1/10

(54) **Active hearing protection system and method**

(71) Applicant: PHONAK AG, 8712 Stäfa (CH)
(72) Inventor: Berg, Christian, 8713 Uerikon (CH)
(74) Representative: Schwan - Schwan - Schorer

(57) **Abstract**

The invention relates to a hearing protection system comprising a first and a second hearing protection device (10), each hearing protection device comprising an active unit (16) comprising an acoustic input transducer (18, 19) for converting ambient sound into input audio signals, and an acoustic output transducer (22) for transforming filtered audio signals into sound perceivable by said user, wherein at least one of said active units comprises an audio signal processing unit (20) for processing said input audio signals into said filtered audio signals; wherein said audio signal processing unit comprises an analyzer module (34) for determining the intensity of said input audio signals separately for a plurality of spectral classes, a judgement module (36) for judging, depending on said determined spectral intensities of said input digital audio signals, which one of a plurality of predetermined criteria is presently fulfilled by the input audio signals, and a filter module (38) having adaptive frequency and time domain filter settings, depending on the judgement made by said judgement module, for producing said filtered audio signals, said judgement being adapted to detect close speech; and wherein said filter settings are selected by said judgement module such that said filter settings provide for a transparent mode if the judgement module judges that no noise is present, for a first attenuation mode if the judgement module judges that noise without close speech is present, and for a second attenuation mode if the judgement module judges that noise with close speech is present.

## Description

The present invention relates to a system and a method for providing active hearing protection to a person.

A large part of the population is exposed to hazardous noise from time to time. This can be at work, whilst traveling, during leisure activities or at home. The exposure can lead to permanent hearing loss, distract people's attention from other hazards or simply cause stress. In order to prevent both accidents and permanent hearing damage, hearing protection devices (HPDs) have been provided in many styles and over many years. It started with the earmuff which is still very relevant and addresses very noisy environments (e.g. airports, construction, shooting) or complex working/communication situations (e.g. fighter pilots). Over the years development of biocompatible soft materials has enabled soft earplugs in different styles and colors as well as recent development of "one fits many" standard semi-soft earplugs in silicon-rubber type materials. For severe situations even the combination of an earmuff and an "in-the-ear" HPD is required to achieve desired attenuation. The physical limitation of hearing protection based on ear worn devices is defined where bone-conduction (body acoustics) becomes dominant at around 40 dB attenuation.

A common disadvantage of the above mentioned HPD styles is wearing discomfort. In case of the earmuffs, they are large which creates difficulties in combination with other head worn gear and they "close off" the ear too much for most applications. The in-the-ear styles mentioned are devices made to fit "the average" ear in one way or the other. Either the fit is provided by softness of the material which leads to undefined device insertion and undefined attenuation, or the fit is provided by standard shaped structures intended to block off the ear canal. In both cases the flat distribution of the individual shape of the outer ear and the ear canal leads to bad fit, pressure points in the ear and undefined positioning of the device.

To address this wearing comfort issue, in-the-ear hearing aid technology has been applied making customized ear molds with passive acoustical filter. These are long lasting devices with good wearing comfort. However, this customization process is traditionally a very manual process creating varying results over time, low reproducibility and the quality is very operator skill dependent.

Customized earplugs are earplugs comprising a hard shell which has an outer surface individually shaped according to the measured inner shape of the user's outer ear and ear canal. Such earplugs presently are primarily used for housing hearing aids. The inner shape of the user's outer ear and ear canal may be measured, for example, by direct laser scanning or by forming an impression. The customized hard shell may be produced by an additive process, such as layer-by-layer laser sintering of a powder material. Customized earplugs are described, for example, in WO 02/071794 A1, US 2003/0133583 A1 or US 6,533,062 B1.

On the other hand, soft earplugs are widely used, in particular also as hearing protection devices. A soft earplug has an outer surface with a standardized shape and is made of a relatively soft material so that the outer surface of the earplug is capable of adapting its shape to the individual inner shape of the user's outer ear and ear canal.

It is commonly known to design hearing protection devices as so-called active hearing protection devices wherein each device is provided with an outer microphone for converting ambient sound into input audio signals, a signal processing unit for processing the input audio signals into output audio signals, and an acoustic output transducer, i.e. speaker or receiver, which converts the audio output signals into sound perceivable by the user when wearing the hearing protection device. Thereby the hearing protection device is provided with a communication function enabling the user to perceive ambient sound signals in a controlled manner even when wearing the hearing protection device in a noisy environment. In order to provide for a selective communication function, i.e. in order to enable the user to perceive, for example, speech while suppressing undesired noise, it is known to provide the audio signal processing unit with the capability of assessing the sound picture and adapting filter and gain settings to the noise level dynamically.

An example of such a system is described in US 4,677,678 which relates to hearing protection devices with an active processing and adaptive circuitry ("Automatic Gain Control" (AGC)), with one AGC taking care of the sound in both ears and with the hearing protection devices being electrically connected for providing a binaural functionality. WO 02/17837 A1 relates to an active hearing protection device which comprises, in addition to the outer microphone, an inner microphone picking up sound at the distal end of the hearing protection device, wherein desired sound is selectively filtered and amplified based on input audio signal analysis.

US 5,027,410 relates to an adaptive digital hearing aid system which digitally classifies the sound input and selects spectral coefficients of the audio signal processing unit according to the result of the sound input classification in order to reduce background noise levels relative to speech and in order to limit the speaker output for avoiding sound signals exceeding the comfort level of the user.

US 4,750,207 relates to an analogue hearing aid system with frequency selective audio signal amplification in response to the sound input in order to provide for a background noise suppression feature.

US 2001/0046304 A1 relates to a headset device for providing external audio signals to the user's ear having a hear-through function for selected audio signals, which is achieved by analyzing the ambient sound for predetermined stored sound signals and by selectively amplifying the ambient sound in this mode over the external audio signals to be provided to the user's ear by the headset.

It is an object of the invention to provide for a hearing protection system which provides for sufficient hearing protection in noisy environments while it, at the same time, provides for a communication function, in particular in order to enable the user to perceive speech, both in high noise environments and in low noise environments. In addition, the hearing protection system should be easy to handle by the user. It is a further object to provide for a corresponding hearing protection method.

These objects are achieved by a hearing protection system as defined in claim 1 and a method as defined in claim 17.

The invention is beneficial in that, by providing for a classification of the actual ambient sound scenario and accordingly selecting a transparent mode, a first attenuation mode without close speech being present and a second attenuation mode with close speech being present, an automatic and appropriate adaptation of the total amplification provided by the active part of the hearing protection system according to the present ambient sound scenario is achieved, whereby both safe hearing protection and a communication function in quiet and noisy environments is achieved while handling of the hearing protection system by the user is kept particularly simple.

Preferred embodiments of the invention are defined in the dependent claims.

In the following, examples of the invention will be illustrated by reference to the attached drawings.

Fig. 1 shows a schematic representation of a hearing protection system according to the invention; and

Fig. 2 shows a schematic representation of the operation modes of the system of Fig. 1.

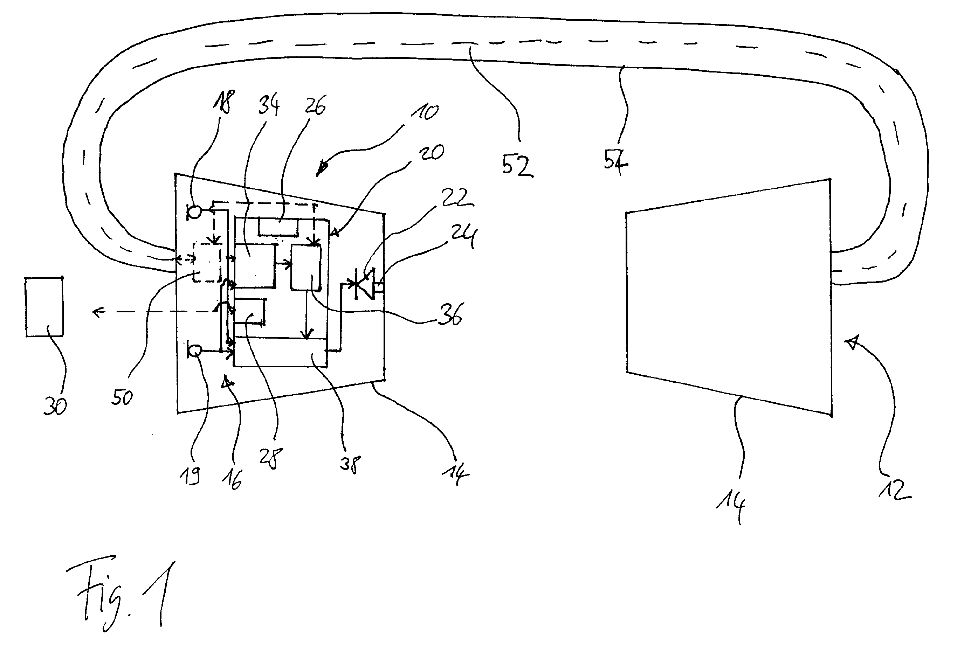

Fig. 1 is a schematic representation of a hearing protection system for a person comprising a hearing protection earplug 10 which is to be worn at least partly within a person's right ear canal and a hearing protection earplug 12 which is to be worn at least partly within the person's left ear canal. In a less preferred embodiment, the earplugs 10, 12 may be replaced by hearing protection devices which are to be worn at the person's right and left ear, respectively, such as an earmuff.

In general, the hearing protection devices according to the invention are adapted to provide for an acoustic attenuation of at least 10 dB averaged over the audible frequency range when worn by the user.

Each hearing protection earplug 10, 12 comprises a shell 14 which is adapted to be worn at least in part in a user's ear canal, i.e. at least a distal portion of the shell is to be inserted into the outer part of the user's ear canal, in order to provide for an acoustic attenuation of at least 10 dB averaged over the audible frequency range when the earplug is worn by the user in order to protect the user from excessive levels of ambient sound. The earplug may comprise an acoustic filter for adjusting the desired total acoustic attenuation or for adjusting the frequency dependent acoustic attenuation.

The shell preferably is a hard shell having an elasticity from shore D85 to D65 and preferably is made of polyamide. In order to achieve optimized fit of the shell within the user's outer ear and ear canal, the shell has an outer surface individually shaped according to the measured shape of the user's outer ear and ear canal, i.e. the shell has an individually customized outer shape. The shape of the user's outer ear and ear canal may be determined by direct three-dimensional scanning of the ear canal and the concha or by producing an impression of the ear canal and the concha which subsequently undergoes scanning. The scanning process may be carried out optically, preferably by laser scanning.

The digital data obtained by the scanning process is then used to create the hard shell by an additive or incremental layer-by-layer build up process. Such processes are also known as "rapid prototyping". A preferred additive build-up process is a layer-by-layer laser sintering process of powder material, preferably polyamide powder. Such processes are also known as "selective laser sintering" (SLS). The basic principle therein is the repeated deposition of a thin layer of material on a surface, with the desired sectional shape then being stabilized, i.e. hardened, by laser action. Other preferred additive layer-by-layer build-up processes are laser stereo-lithography or photo-polymerization. An overview regarding additive layer-by-layer build-up processes for producing customized shells for hearing aids can be found, for example, in US 2003/0133583 A1 or US 6,533,062 B1.

In general, the invention relates to active hearing protection devices, i.e. hearing protection devices which do not only provide for an acoustic attenuation of ambient sound in order to protect the user's hearing, but in addition comprise means for electro-acoustically by-passing this acoustic attenuation function in order to provide the user with a communication function, e.g. in order to enable the user to perceive speech signals, even when wearing the hearing protection devices in a noisy environment.

To this end, each earplug 10, 12 is provided with an active unit 16 which comprises a microphone 18 for converting ambient sound impinging on the proximal, i.e. outer, end of the earplug 10, 12 into input audio signals, an audio signal processing unit 20 for processing the input audio signals provided by the microphone 18 into filtered audio signals and an acoustic output transducer, i.e. speaker, 22 for converting the filtered audio signals provided by the audio signal processing unit 20 into sound which is perceivable by the user's ear via a sound output opening 24 provided at the distal end of each earplug 10, 12. In practice, the audio signal processing unit 20 may be realized by a digital signal processor which includes a memory 26 for storing programs and data. Consequently, the active unit 16 in addition comprises an analogue-to-digital converter between the microphone 18 and the audio signal processing unit 20 and a digital-to-analogue converter between the audio signal processing unit 20 and the speaker 22 (these converters are not shown in Fig. 1). Further, the audio signal processing unit 20 is provided with an interface 28 for temporarily connecting with a remote unit 30 via a wireless or wired data connection 32 for uploading programs and data into the audio signal processing unit 20, i.e. the program/data memory 26. Such data may include user specific audio data, such as an existing hearing loss, in order to individually configure the audio signal processing unit for optimizing performance. The active unit 16 further comprises a battery which is not shown in Fig. 1.

According to the invention, the audio signal processing unit 20 is designed to enable, on the one hand, selective perception of desired sound signals, such as speech signals, both in low noise and high noise environments while, on the other hand, protection of the user's hearing from excessive ambient sound levels is ensured. To this end, the audio signal processing unit 20 comprises an analyzer module 34 for determining the intensity of the input audio signals provided by the microphone 18 separately for a plurality of spectral classes, a judgement module 36 for judging, depending on the spectral intensities of the input audio signals determined by the analyzer module 34, which one of a plurality of predetermined criteria is presently fulfilled by the input audio signals, and a filter module 38 having adaptive frequency and time domain filter settings for producing the filtered audio signals for the speaker 22, with the filter settings of the filter module 38 depending on the judgement made by the judgement module 36. The judgement module 36 is capable of detecting close speech, i.e. speech signals originating from a person which is presently located close to the user's ear. "Close" in this respect is to be understood as a distance of less than 3 m, preferably less than 1.5 m.

The filter settings of the filter module 38 are selected by the judgement module 36 such that the filter settings provide at least for the following modes: a transparent mode if the judgement module 36 judges that no noise is present (for example, the noise levels are below predefined threshold values); a first attenuation mode if the judgement module 36 judges that noise without close speech is present (for example, the noise levels exceed the predefined threshold values); and a second attenuation mode if the judgement module 36 judges that noise with close speech is present (i.e. in addition to noise also close speech signals are detected by the judgement module 36).

The criteria used by the judgement module 36 for judging in which of these modes the filter module 38 is to be operated may be individually defined according to, for example, previous measurements of the user's hearing capability.

Preferably, the judgement module 36 is designed to determine, in order to make the filter settings mode judgement, whether for each spectral class the intensity has fallen within a given range for a given minimum time interval, with the ranges and time intervals depending on the respective filter settings mode. Further, the judgement module 36 may be designed to analyze, in order to make the filter settings mode judgement, at least one audio signal parameter selected from the group consisting of attack time, release time, signal dynamics, spectral content, spectral dynamics, and signal history. Consequently, in preferred embodiments of the invention the judgement module 36 will not only consider the present levels in the different spectral classes provided by the analyzer module 34 but in addition will also consider the variation of these levels with time in order to achieve an optimized judgement of the present audio situation.

The judgement module 36 and the filter module 38 may be designed such that in the transparent mode the frequency averaged amplification provided by the active unit 16 has a first value, in the first attenuation mode the frequency averaged amplification provided by the active unit 16 has a second value which is lower than the first value, and in the second attenuation mode the frequency averaged amplification provided by the active unit 16 has a third value lower than the first value and speech frequencies, i.e. frequencies preferably between 500 Hz and 5 kHz, are selectively amplified over other frequencies.

Preferably not only the judgement criteria of the judgement module 36 but also the filter settings of the filter module 38 in each mode are individually defined, for example, taking into account the user's hearing capability including hearing loss, in order to individually optimize the performance of the active unit 16.

In the transparent mode the first amplification value may be selected such that the total amplification, i.e. the amplification including the acoustic attenuation provided by the shell 14, is between -10 dB and 0 dB. Preferably, in the first attenuation mode the second amplification value is selected such that the total amplification, i.e. including the acoustic attenuation provided by the shell 14, is less than -10 dB.

In order to allow the judgement module 36 to detect close speech, each earplug 10, 12 is provided with a second microphone 19 which is located spaced apart from the microphone 18 in order to enable the judgement module 36 to assign angular directions to the input audio signals by analyzing phases of the input audio signals, i.e. by determining the phase difference of the input audio signal provided by the microphone 18 and the input audio signal provided by the microphone 19. Further, the judgement module 36 analyzes the power spectrum of the input audio signals for these angular directions and determines, by comparison with a predefined speech judgement condition, whether angularly directed speech signals are present in the input audio signals. Preferably, such speech judgement condition includes intensity limits for a plurality of frequency bands and a threshold value for the inhomogeneity of the angular distribution of speech signals.

In addition or alternatively to the above described approach of determining the angular distribution of speech signals the judgement module 36 may be adapted to determine, by time, frequency and intensity dependent analysis of the input audio signals for determining the presence of spatially reflected signals and by comparison with a predefined speech judgement condition, whether reflected signals within a pre-defined speech frequency range, preferably 500 Hz to 5 kHz, and with a delay time above a pre-defined limit are present in the input audio signals, with the speech judgement condition including values for this speech frequency range and for this delay time limit.

Examples for methods and devices for achieving ambient sound classification, and in particular also the distinction between undesired noise signals and desired sound signals, are described, for example, in US 5,027,410, wherein also selective filter setting of the audio signal processing unit according to the result of the sound classification is described.

Examples and examples of methods and devices for localizing speech sources in background noise are described, for example, in EP 1 320 281 A1, WO 00/68703, EP 1 005 783 B1, US 6,522,756 B1, WO 00/33634, WO 01/60112 and EP 0 802 699 A2. In these documents it is also described how detected speech signals can be selectively amplified over background noise.

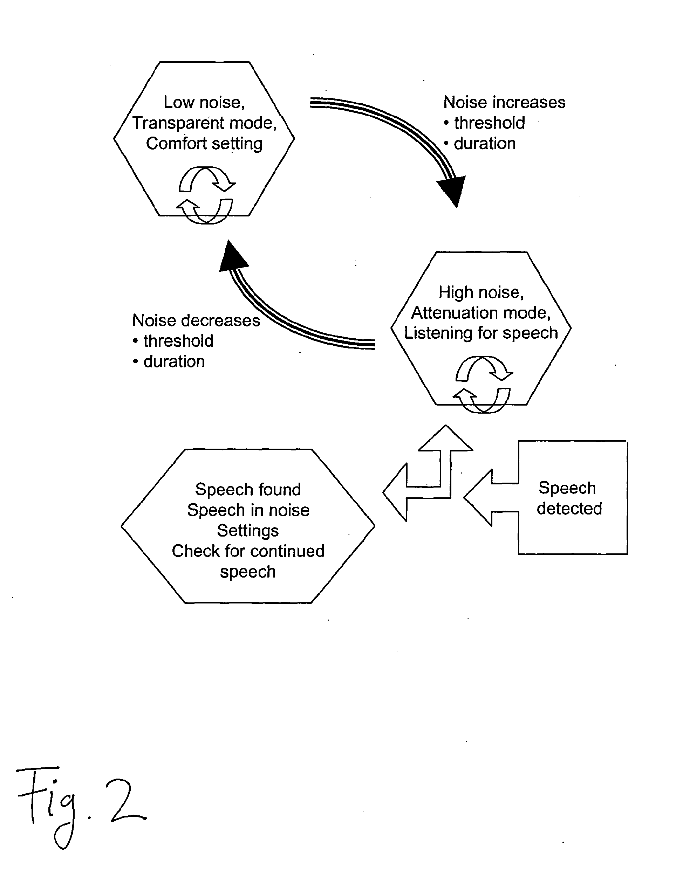

Fig. 2 is a schematic representation of an example for the operation of an active hearing protection system according to the invention. It is assumed that the active unit 16 starts in the transparent mode, i.e. the system is worn in a sound environment with low noise levels. In this mode, the main purpose of the active unit 16 is to more or less bypass the (mechanical) acoustic attenuation provided by the earplugs 10, 12 when having been inserted into the user's ear canals in order to enable the user to perceive the ambient sound, in particular speech signals, in a close-to-natural manner. In this mode, the settings of the filter module 38 are selected such that maximum hearing comfort is achieved ("comfort setting").

Once the noise increases in such a manner that the judgement module 36 judges that the "high noise" criteria are fulfilled, e.g. that threshold levels in the noise frequency bands are exceeded for a given minimum duration, the settings of the filter module 38 are changed into the first attenuation mode in order to protect the user from excessive noise levels, i.e. the amplification provided by the active unit 16 is significantly reduced or even completely eliminated in order to provide for a maximum acoustic attenuation by the earplugs 10, 12. Once the judgement module 36 finds that the noise has decreased in such a manner that the "high noise" criteria are no longer fulfilled, the settings of the filter module 38 will be changed to those for the transparent mode again.

However, in the first attenuation mode the judgement module 36 continuously searches for close speech signals. If such close speech signals are found, the system will enter the second attenuation mode and the settings of the filter module 38 will be changed in such a manner that, while noise signals are still suppressed as in the first attenuation mode, the detected speech signals are selectively amplified over the noise signals by selectively amplifying only speech frequencies and/or selectively amplifying audio signals only from angular directions in which a speech source has been detected. If no close speech signals are detected any longer, the system will return to the first attenuation mode.

In order to enhance comfort of the hearing protection system, soft fading between the various modes may be applied. In general, the change between the various modes of operation occurs fully automatically without any manipulation by the user being necessary.

The evaluation of the noise situation should occur essentially permanently, for example, at least once or twice per second, in order to ensure a sufficiently fast response to changes in the noise/communication situation.

In general, additional operation modes are possible. For example, the judgement module 36 may be adapted to detect acoustic alarm signals in order to change, once an acoustic alarm signal has been detected, the settings of the filter module 38 in such a manner that acoustic perception of this alarm signal by the user is enabled, for example, by selective amplification of the alarm signal frequencies over background noise frequencies. Such a mode could be present as a third attenuation mode.

Similar to the second attenuation mode, the active unit 16 would return to the first attenuation mode once the alarm signal is no longer detected by the judgement module 36.

Preferably, the active unit 16 of each of the earplugs 10, 12 is provided with one of the signal processing units 20.

In general, the hearing protection system may be designed as a binaural system (see elements shown in dashed lines in Fig. 1). In this case, each earplug 10, 12 may be provided with a binaural communication unit 50 which communicates bidirectionally with its counterpart in the other earplug via a communication link 52 which may be integrated into means for mechanically connecting the earplugs 10, 12, such as a cord 54, for preventing loss of the earplugs 10, 12. In addition, each binaural communication unit 50 communicates with the respective audio signal processing unit 20, in particular the respective judgement unit 36.

In such a system the audio signal processing units 20, and in particular the judgement units 36 of the right earplug 10 and the left earplug 12, are enabled to communicate with each other in order to improve the accuracy of the judgement of the speech/noise situations and the accuracy of the detection of close speech directions.

## Claims

1. A hearing protection system comprising a first hearing protection device (10) to be worn at a person's right outer ear and/or at least in part in a person's right ear canal and a second hearing protection device (12) to be worn at said person's left outer ear and/or at least in part in said person's left ear canal, each hearing protection device comprising an active unit (16) comprising an acoustic input transducer (18, 19) for converting ambient sound into input audio signals, and an acoustic output transducer (22) for transforming filtered audio signals into sound perceivable by said user, wherein at least one of said active units comprises an audio signal processing unit (20) for processing said input audio signals into said filtered audio signals;
wherein each hearing protection device is adapted to provide for an acoustic attenuation of at least 10 dB averaged over the audible frequency range when worn by the person;
wherein said audio signal processing unit comprises an analyzer module (34) for determining the intensity of said input audio signals separately for a plurality of spectral classes, a judgement module (36) for judging, depending on said determined spectral intensities of said input digital audio signals, which one of a plurality of predetermined criteria is presently fulfilled by the input audio signals, and a filter module (38) having adaptive frequency and time domain filter settings, depending on the judgement made by said judgement module, for producing said filtered audio signals, said judgement being adapted to detect close speech; and
wherein said filter settings are selected by said judgement module such that said filter settings provide for a transparent mode if the judgement module judges that no noise is present, for a first attenuation mode if the judgement module judges that noise without close speech is present, and for a second attenuation mode if the judgement module judges that noise with close speech is present.

2. The hearing protection system of claim 1, wherein in said transparent mode the frequency averaged amplification provided by said active unit (16) has a first value, in said first attenuation mode the frequency averaged amplification provided by said active unit has a second value lower than the first value, and in said second attenuation mode the frequency averaged amplification provided by said active unit has a third value lower than the first value and speech frequencies are selectively amplified.

3. The hearing protection system of one of claims 1 or 2, wherein each active unit (16) comprises one of said audio signal processing units (20).

4. The hearing protection system of one of the preceding claims, wherein said filter settings in each mode are individually defined.

5. The hearing protection system of one of the preceding claims, wherein said judgement module (36), in order to make said filter settings mode judgement, is adapted to determine whether for each spectral class the intensity has fallen within a given range for a given minimum time interval, said ranges and time intervals depending on the respective filter settings mode.

6. The hearing protection system of one of the preceding claims, wherein said judgement module (36) is adapted, in order to make said filter settings mode judgement, to analyze at least one audio signal parameter selected from the group consisting of attack time, release time, signal dynamics, spectral content, spectral dynamics, and signal history.

7. The hearing protection system of claim 2, wherein said first amplification value is selected such that the total amplification, including said acoustic attenuation, provided by each hearing protection device (10, 12) is between -10 dB and 0 dB.

8. The hearing protection system of one of claims 2 or 7, wherein said second amplification value is selected such that the total amplification, including said acoustic attenuation, provided by each hearing protection device (10, 12) is less than- 10 dB.

9. The hearing protection system of claim 2, wherein said speech frequencies are between 500 Hz and 5 kHz

10. The hearing protection system of one of the preceding claims, wherein said hearing protection devices are designed as earplugs (10, 12) having a shell (14).

11. The hearing protection system of one of the preceding claims, wherein the outer surface of said shell (14) is individually shaped according to the measured inner shape of the person's outer ear and ear canal.

12. The hearing protection system of claim 11, wherein said shell (14) is a hard shell with an elasticity of from shore D85 to shore D65.

13. The hearing protection system of one of the preceding claims, wherein said judgement module (36) is adapted to assign, by analyzing phases of said input audio signals, angular directions to said input audio signals and to judge, by analyzing the power spectrum of said audio input signals for said angular directions and by comparison with a speech judgement condition, whether angularly directed speech signals are present in said audio input signals.

14. The hearing protection system of claim 13, wherein said speech judgement condition includes intensity limits for a plurality of frequency bands and a threshold for the inhomogeneity of the angular distribution of speech signals, and wherein said judgement module (36) judges that close speech is present if said intensity limits and said threshold value are exceeded.

15. The hearing protection system of one of claims 13 or 14, wherein each active unit (16) includes at least one additional acoustic input transducer (19).

16. The hearing protection system of one of the preceding claims, wherein said judgement module (36) is adapted to determine, by time, frequency and intensity dependent analysis of said input audio signals for determining the presence of spatially reflected signals and by comparison with a speech judgement condition, whether reflected signals within a defined speech frequency range and with a delay time above a defined limit are present in said audio input signals, said speech judgement condition including values for said speech frequency range and for said delay time limit.

17. A method for providing active hearing protection to a person, comprising:
providing a first hearing protection device (10) at said person's right outer ear and/or at least in part in said person's right ear canal and a second hearing protection device (12) at said person's left outer ear and/or at least in part in said person's left ear canal in such a manner that an acoustic attenuation of at least 10 dB averaged over the audible frequency range is achieved;
converting, in each hearing protection device, ambient sounds into input audio signals and converting filtered audio signals into sound perceivable by said person, wherein said input audio signals are processed into said filtered audio signals in at least one of said hearing protection devices;
determining, in at least one of said hearing protection devices, the intensity of said input audio signals separately for a plurality of spectral classes; judging, depending on said determined spectral intensities of said input audio signals, which one of a plurality of predetermined criteria is presently fulfilled by the input audio signals; and specifically selecting, depending on the judgement made, frequency and time domain filter settings for producing said filtered audio signals;
wherein said filter settings are selected such that said filter settings provide for a transparent mode if the judgement made is that no noise is present, for a first attenuation mode if the judgement made is that noise without close speech is present, and for a second attenuation mode if the judgement made is that noise with close speech is present.

18. The method of claim 17, wherein in said transparent mode the frequency averaged amplification has a first value, in said first attenuation mode the frequency averaged amplification has a second value lower than the first value, and in said second attenuation mode the frequency averaged amplification has a third value lower than the first value and speech frequencies are selectively amplified.

19. The method of one of claims 17 or 18, wherein said input audio signals are processed into said filtered audio signals in both of said hearing protection devices (10, 12).

20. The method of one of claims 17 to 19, wherein said filter settings in each mode are individually defined.

21. The method of one of claims 17 to 20, further comprising: determining, in order to make said filter settings mode judgement, whether for each spectral class the intensity has fallen within a given range for a given minimum time interval, said ranges and time intervals depending on the respective filter settings mode.

22. The method of one of claims 17 to 21, further comprising: analyzing, in order to make said filter settings mode judgement, at least one audio signal parameter selected from the group consisting of attack time, release time, signal dynamics, spectral content, spectral dynamics, and signal history.

23. The method of claim 18, wherein said first amplification value is selected such that the total amplification, including said acoustic attenuation, provided by each hearing protection device (10, 12) is between -10 dB and 0 dB.

24. The method of one of claims 18 and 23, wherein said second amplification value is selected such that the total amplification, including said acoustic attenuation, provided by each hearing protection device is less than -10 dB.

25. The method of claim 18, wherein said speech frequencies are between 500 Hz and 5 kHz.

26. The method of one of claims 17 to 25, further comprising, in order to detect close speech, assigning, by analyzing phases of said input audio signals, angular directions to said input audio signals and judging, by analyzing the power spectrum of said audio input signals for said angular directions and by comparison with a speech judgement condition, whether angularly directed speech signals are present in said audio input signals.

27. The method of claim 26, wherein said speech judgement condition includes intensity limits for a plurality of frequency bands and a threshold for the inhomogeneity of the angular distribution of speech signals, and wherein it is judged that close speech is present if said intensity limits and said threshold value are exceeded.

28. The method of one of claims 17 to 27, further comprising, in order to detect close speech, determining, by frequency dependent analysis of said input audio signals for determining the presence of spatially reflected signals and by comparison with a speech judgement condition, whether reflected signals within a defined speech frequency range and with a delay time above a defined limit are present in said audio input signals, said speech judgement condition including values for said speech frequency range and for said delay time limit.
